Europäisches Patentamt

European Patent Office

Office européen des brevets

(19)

(11) Veröffentlichungsnummer: **0 252 064 B1**

## (12) EUROPÄISCHE PATENTSCHRIFT

(45) Veröffentlichungstag der Patentschrift: **25.11.92**

(21) Anmeldenummer: **87890135.4**

(22) Anmeldetag: **15.06.87**

Die Akte enthält technische Angaben, die nach dem Eingang der Anmeldung eingereicht wurden und die nicht in dieser Patentschrift enthalten sind.

(51) Int. Cl.⁵: **A61K 39/104**, A61K 39/40

(54) **Gegen Pseudomonas aeruginosa Infektionen wirksame Präparationen und Verfahren zu ihrer Herstellung.**

(30) Priorität: **24.06.86 AT 1716/86**

(43) Veröffentlichungstag der Anmeldung:
**07.01.88 Patentblatt 88/01**

(45) Bekanntmachung des Hinweises auf die Patenterteilung:
**25.11.92 Patentblatt 92/48**

(84) Benannte Vertragsstaaten:
**AT BE CH DE ES FR GB IT LI LU NL SE**

(56) Entgegenhaltungen:
**WO-A-86/03974**

(73) Patentinhaber: **IMMUNO Aktiengesellschaft**
**Industriestrasse 67**
**A-1221 Wien(AT)**

(72) Erfinder: **Dorner, Friedrich, Prof. Dr.**
**Peterlinigasse 17**
**A-1230 Wien(AT)**
Erfinder: **Eibl, Johann, Dr.**
**Gustav Tschermakgasse 2**
**A-1180 Wien(AT)**

(74) Vertreter: **Wolfram, Gustav, Dipl.-Ing.**
**Patentanwälte Sonn, Pawloy, Weinzinger &**
**Wolfram, Riemergasse 14**
**A-1010 Wien(AT)**

EP 0 252 064 B1

ABSTRACTS OF THE ANNUAL MEETING OF THE AMERICAN SOCIETY FOR MICROBIOLOGY, Band 85, Nr. O, 1985, Seite 12, Nr. A69; I.A. HOLDER et al.: "Immunization using divalent Flagella preparations: protection of burned, Pseudomonas Seruginosa infected mice"

ABSTRACTS OF THE ANNUAL MEETING OF THE AMERICAN SOCIETY FOR MICROBIOLOGY, Band 85, Nr. O, 1985, Seite 42, Nr. B143; D. DRAKE et al.: "Passive protection with Flagellar antiserum against Pseudomonas aeruginosa infection in compromised mice"

ABSTRACTS OF THE ANNUAL MEETING OF THE AMERICAN SOCIETY FOR MICROBIOLOGY, Band 81, 1981, Seite 17, Nr. B17; T.C. MONTIE et al.: "Proctection of burned, Pseudomonas infected mice using Flagellar preparations"

INFECTION AND IMMUNITY, Band 35, Nr. 1, Januar 1982, Seiten 276-288; T.C. MONTIE et al.: "Flagellar preparations from Pseudomonas aeruginosa: Isolation and characterization"

INFECTION AND IMMUNITY, Band 49, Nr. 3, September 1985, Seiten 770-774; J.S. ALLISON et al.: "Electrophoretic separation and molecular weight characterization of Pseudomonas aeruginosa H-antigen flagellins"

INFECTION AND IMMUNITY, Band 35, Nr. 1, Januar 1982, Seiten 276-280; I.A. HOLDER et al.: "Flagellar preparations from Pseudomonas aeruginosa: Animal protection studies"

Med. Microbiol. Immunol. 168 (1980) 217-226

Discovery and Isolation of Microbiol. Products; Ed. M S Verrall, Ellis Horwood Ltd. 1985

CIBA Foundation Symposium 80, Pitman Medical (1981) 188, 199, 200

Zbl. Bakt. Hyg., I Abt. Orig. A 246 (1980) 363-372

**Beschreibung**

Die Erfindung betrifft gegen Pseudomonas aeruginosa Infektionen wirksame Präparationen. Im besondern betrifft die Erfindung Vakzine, die protektive Pseudomonas aeruginosa Flagella(H)-Antigene enthalten und zur aktiven Immunisierung bestimmt sind, sowie Immunglobulin-G-hältige, gegen Bakterium Pseudomonas aeruginosa-Infektionen wirksame Präparationen, die zum passiven Schutz bestimmt sind.

Bakterium Pseudomonas aeruginosa ist ein opportunistischer, pathogener Keim, der häufig bei Krankenhausinfektionen auftritt, hauptsächlich bei Patienten mit geschwächter Immunabwehr, wie bei Verbrennungspatienten, bei Personen, die an cystischer Fibrose leiden oder organische Fehlfunktionen aufweisen, und bei Tumorpatienten. Antibiotika sind gegen Pseudomonasinfektionen infolge des Auftretens von Resistenzen nur beschränkt wirksam, weshalb man bemüht ist, immunologische Methoden zur Bekämpfung von Infektionen durch Pseudomonas aeruginosa zu finden.

Infektionen können durch eine Vielzahl von Stämmen ausgelöst werden, die O-Gruppenantigene und H-Antigene produzieren. Gemäß dem H-Antigenschema nach Ansorg (Zbl. Bakt. Hyg. I. Abt. Orig. A 242, 228 - 238 (1978) wird unter Verwendung der indirekten Immunfluoreszenztechnik bei Pseudomonas aeruginosa ein komplexes Flagella(H)-Antigen a mit den Partialantigenen $a_0$, $a_1$, $a_2$, $a_3$, $a_4$ und ein uniformes Flagella-(H)-Antigen b differenziert. Die Partialfaktoren $a_0$-$a_4$ sind unabhängige Determinanten, so daß ein flagellares Antigenschema mit mehreren H-Typen resultiert. O-Gruppen und H-Typ zeigen freie Kombinationen.

Die zur Bereitung von Pseudomonas aeruginosa-Bakterienkulturen verwendeten Stämme und die produzierten Antigene sind in der folgenden Tabelle 1 angeführt.

## Tabelle 1

|   | Stamm | H-Typ | |
|---|-------|-------|---|
| 1 | 170001 | – | b |
|   | M-2 | – | b |
| 2 | 5142 | – | $a_0$ |
| 3 | 5940 | – | $a_0$, $a_2$ |
| 4 | 5939 | – | $a_0$, $a_3$ |
| 5 | 5933 | – | $a_0$, $a_1$, $a_2$ |
|   | 1210 | – | $a_0$, $a_1$, $a_2$ |
|   | 16990 | – | $a_0$, $a_1$, $a_2$ |
| 6 | 170018 | – | $a_0$, $a_3$, $a_4$ |

Isolierte Filamente der Flagellen-Antigene, die durch Schütteln, Homogenisieren und anschließendes Zentrifugieren gewonnen werden können (R. Ansorg, W. Schmitt, Med. Microbiol. Immunol. (1980) 168: 217-226), bestehen aus Flagellen und Flagellenbruchstücken, vereint in einem Komplex bestehend aus Lipopolysacchariden (LPS) und Verunreinigungen aus dem Nährmedium; solche Präparationen sind naturgemäß pyrogen und ungeeignet für die Anwendung am Menschen.

In der PCT-Veröffentlichung WO 86/03974 ist die Struktur von nach einem Reinigungsverfahren gewonnenen polydispersen nativen Pseudomonas-Flagella(H)-Antigenen (FAg) beschrieben, die frei sind von pyrogenen Substanzen. Sie bestehen aus monomeren Bestandteilen, wobei jeder monomere Bestandteil

a) folgende Aminosäuren: Asparaginsäure bzw. Asparagin (Asp/Asn), Threonin (Thr), Serin (Ser), Glutaminsäure bzw. Glutamin (Glu/Gln), Glycin (Gly), Alanin (Ala), Valin (Val), Isoleucin (Ile), Leucin (Leu), Tyrosin (Tyr), Phenylalanin (Phe), Lysin (Lys), Arginin (Arg) und gegebenenfalls Tryptophan (Trp) enthält,

b) die N-terminale Aminosäuresequenz Ala-Leu-Thr-Val-AsnThr-Asn-Ile-Ala- aufweist,

c) ein Molekulargewicht zwischen 43.500 und 53.050 aufweist und

d) frei ist von Prolin (Pro), Methionin (Met), Halb-Cystin (1/2 Cys) und Histidin (His).

Weiterhin sind die einzelnen Flagella(H)-Antigentypen dadurch gekennzeichnet, daß die monomeren Formen die Aminosäuren Asparagin/-säure, Threonin, Serin, Glutamin/säure, Glycin, Alanin, Valin, Isoleucin, Leucin, Tyrosin, Phenylalanin, Lysin und Arginin in bestimmten Verhältnissen zueinander enthalten. In Tabelle 2 sind diese Verhältniszahlen und die Molekulargewichte der monomeren Flagella(H)-Antigene angeführt.

**Tabelle 2**

**Anzahl AS/Molekül Flagellin**

**Stämme mit zugehörigem H-Serotyp**

| Aminosäuren | 5940 | 1210 | M-2 | 5142 | 5939 | 170018 |
|---|---|---|---|---|---|---|
| Asparaginsäure | 68 | 76 | 74 | 74 | 69 | 64 |
| Threonin | 41 | 44 | 48 | 50 | 35 | 33 |
| Serin | 37 | 40 | 48 | 49 | 38 | 35 |
| Glutaminsäure | 46 | 52 | 49 | 49 | 44 | 42 |
| Glycin | 44 | 50 | 51 | 49 | 47 | 44 |
| Alanin | 73 | 81 | 91 | 89 | 73 | 68 |
| Valin | 29 | 32 | 38 | 37 | 30 | 29 |
| Isoleucin | 29 | 32 | 30 | 29 | 30 | 29 |
| Leucin | 37 | 41 | 43 | 44 | 60 | 37 |
| Tyrosin | 3 | 4 | 4 | 5 | 3 | 3 |
| Phenylalanin | 10 | 12 | 13 | 14 | 12 | 10 |
| Lysin | 16 | 20 | 18 | 17 | 21 | 19 |
| Arginin | 16 | 18 | 18 | 16 | 16 | 15 |
| W = | 449 | 502 | 525 | 522 | 478 | 450 |
| MG = | 45.900 | 51.250 | 53.050 | 52.720 | 46.700 | 43.500 |

Gemäß den Angaben in der PCT Veröffentlichung WO 86/03974 werden zur Gewinnung der polydispersen nativen Pseudomonas aeruginosa Flagella(H)-Antigene Bakterienkulturen in Minimalmedium angezüchtet und anschließend mit einem Detergens behandelt, wonach die Flagella(H)-Antigene aus der Kultur abgetrennt werden. Dabei kann die Bakterienkultur entweder vor der Detergensbehandlung desintegriert und einem Scherprozeß unterworfen werden oder in Gegenwart des Detergens den Scherkräften unterworfen werden. Durch den Zusatz von Detergens wird die Trennbarkeit des Antigens von der Bakterienmasse bewirkt; die sich dann in Lösung befindlichen Antigene können abgetrennt werden.

Anschließend können die, wie oben beschrieben, von der Bakterienmasse abgetrennten Antigene durch eine chromatographische Reinigung von anhaftenden Verunreinigungen wie Lipopolysacchariden, Nucleinsäuren, Salzen, Polysacchariden u.a. befreit werden. Das noch vorhandene Detergens kann durch einen weiteren säulenchromatographischen Reinigungsschritt entfernt werden.

Es war bisher nicht bekannt, ob diese Antigene protektive Wirkung haben oder nicht. Es wurde nun gefunden, daß allen Flagella(H)-Antigenen der in der PCT Veröffentlichung WO 86/03974 beschriebenen Typen protektive Wirkung zukommt, so daß die Herstellung bzw. Bereitstellung von monovalenten und polyvalenten Vaxzinen möglich ist. Diese Aufgabe liegt der vorliegenden Erfindung zugrunde.

Im besondern ist es ein Ziel der Erfindung, eine aktive Immunprophylaxe gegen Infektionen mit Pseudomonas aeruginosa zu ermöglichen, insbesondere für Personengruppen, die ein hohes Risiko haben, Verbrennungen zu erleiden, z.B. für Angehörige von Feuerwehren oder militärisches Personal oder Patienten, die mit immunsupprimierenden Medikamenten behandelt werden sollen.

Eine weitere Aufgabe der Erfindung besteht darin, Immunglobulin G-hältige Präparationen zur Verfügung zu stellen, die zur passiven Immunisierung bei Pseudomonas aeruginosa-Infektionen geeignet sind.

Die erfindungsgemäßen Vakzine sind dadurch gekennzeichnet, daß sie protektive Flagella(H)-Antigene vom H-Serotyp a bzw. b enthalten, welche aus monomeren Bestandteilen bestehen, wobei jeder monomere Bestandteil

a) folgende Aminosäuren: Asparaginsäure (Asp), Threonin (Thr), Serin (Ser), Glutaminsäure (Glu), Glycin (Gly), Alanin (Ala), Valin (Val), Isoleucin (Ile), Leucin (Leu), Tyrosin (Tyr), Phenylalanin (Phe), Lysin (Lys), Arginin (Arg) und gegebenenfalls Tryptophan (Trp) und Methionin (Met) enthält,
b) die N-terminale Aminosäuresequenz Alanin (Ala) - Leucin (Leu) - Threonin (Thr) - Valin (Val) - Asparagin (Asn) - Threonin (Thr) - Asparagin (Asn) - Isoleucin (Ile) - Alanin (Ala) - aufweist,
c) ein Molekulargewicht zwischen 43.500 und 53.050 aufweist und
d) frei ist von Prolin, Halb-Cystin und Histidin und daß sie frei sind von pyrogenen Substanzen, festgestellt durch Pyrogentests mit drei Kaninchen durch i.v.-Injektion von 1 ml Vakzinlösung/kg Körpergewicht mit 20 $\mu$g/ml Protein, wobei die Summe der drei Einzelwerte kleiner als 1,15 $°$C ist.

Im einzelnen werden erfindungsgemäß sechs spezifische H-Serotypen als Komponenten von Vakzinen charakterisiert, u.zw.

- Flagella(H)-Antigene vom H-Serotyp $a_0$, $a_3$ und $a_4$, welche Antigene aus monomeren Bestandteilen bestehen, die die Aminosäuren: Asparaginsäure, Threonin, Serin, Glutaminsäure, Glycin, Alanin, Valin, Isoleucin, Leucin, Tyrosin, Phenylalanin, Lysin und Arginin im Verhältnis 64 : 33 : 35 : 42 : 44 : 68 : 29 : 29 : 37 : 3 : 10 : 19 : 15 enthalten und ein Molekulargewicht von 43.500 aufweisen;
- Flagella(H)-Antigene vom H-Serotyp $a_0$, $a_3$, welche Antigene aus monomeren Bestandteilen bestehen, die die Aminosäuren: Asparaginsäure, Threonin, Serin, Glutaminsäure, Glycin, Alanin, Valin, Isoleucin, Leucin, Tyrosin, Phenylalanin, Lysin und Arginin im Verhältnis 69 : 35 : 38 : 44 : 47 : 73 : 30 : 30 : 60 : 3 : 12 : 21 : 16 enthalten und ein Molekulargewicht von 46.700 aufweisen;
- Flagella(H)-Antigene vom H-Serotyp $a_0$, welche Antigene aus monomeren Bestandteilen bestehen, die die Aminosäuren: Asparaginsäure, Threonin, Serin, Glutaminsäure, Glycin, Alanin, Valin, Isoleucin, Leucin, Tyrosin, Phenylalanin, Lysin und Arginin im Verhältnis 74 : 50 : 49 : 49 : 49 : 89 : 37 : 29 : 44 : 5 : 14 : 17 : 16 enthalten und ein Molekulargewicht von 52.720 aufweisen;
- Flagella(H)-Antigene vom H-Serotyp b, welche Antigene aus monomeren Bestandteilen bestehen, die die Aminosäuren: Asparaginsäure, Threonin, Serin, Glutaminsäure, Glycin, Alanin, Valin, Isoleucin, Leucin, Tyrosin, Phenylalanin, Lysin und Arginin im Verhältnis 74 : 48 : 48 : 49 : 51 : 91 : 38 : 30 : 43 : 4 : 13 : 18 : 18 enthalten und ein Molekulargewicht von 53.050 aufweisen;
- Flagella(H)-Antigene vom H-Serotyp $a_0$, $a_1$, $a_2$, welche Antigene aus monomeren Bestandteilen bestehen, die die Aminosäuren: Asparaginsäure, Threonin, Serin, Glutaminsäure, Glycin, Alanin, Valin, Isoleucin, Leucin, Tyrosin, Phenylalanin, Lysin und Arginin im Verhältnis 76 : 44 : 40 : 52 : 50 : 81 : 32 : 32 : 41 : 4 : 12 : 20 : 18 enthalten und ein Molekulargewicht von 51.250 aufweisen;
- Flagella(H)-Antigene vom H-Serotyp $a_0$, $a_2$, welche Antigene aus monomeren Bestandteilen bestehen, die die Aminosäuren: Asparaginsäure, Threonin, Serin, Glutaminsäure, Glycin, Alanin, Valin, Isoleucin, Leucin, Tyrosin, Phenylalanin, Lysin und Arginin im Verhältnis 68 : 41 : 37 : 46 : 44 : 73 : 29 : 29 : 37 : 3 : 10 : 16 : 16 enthalten und ein Molekulargewicht von 45.900 aufweisen.

Ein Verfahren zur Herstellung der erfindungsgemäßen Vakzine ist dadurch gekennzeichnet, daß Kulturen von Pseudomonas aeruginosa mit einem Detergens behandelt, einem Scherprozeß unterworfen, um Flagella(H)-Antigene abzutrennen, und das abgetrennte Antigen chromatographisch gereinigt, sterilfiltriert, gegebenenfalls mit einem Adjuvans, wie Al(OH)$_3$ vermischt, gewünschtenfalls mit einem Präservans, wie

5

Merthiolat (Warenzeichen), versetzt und in eine galenische Zubereitung formuliert wird.

Weiters umfaßt die Erfindung Immunglobulin G-hältige, gegen Bakterium Pseudmonas aeruginosa-Infektionen wirksame Präparationen zur prophylaktischen und therapeutischen Anwendung, welche dadurch gekennzeichnet sind, daß sie aus dem Blutplasma von mit den genannten protektiven Flagella(H)-Antigenen immunisierten menschlichen Spendern bzw. Säugetieren gewonnene Flagella(H)-Antikörper enthalten.

Diese Flagella(H)-Antikörper besitzen eine motilitätshemmende Wirkung sowie die Fähigkeit einer erhöhten Phagocytose und einer erhöhten intrazellulären Abtötung von Pseudomonas aeruginosa-Bakterien.

Nach bevorzugten Ausführungsformen können diese Immunglobulin G-hältigen, gegen Bakterium Pseudomonas aeruginosa-Infektionen wirksamen Präparationen dadurch hergestellt werden, daß Plasma von mit den genannten protektiven Flagella(H)-Antigenen immunisierten menschlichen Spendern bzw. Säugetieren mit Proteinfällungsmitteln, wie Ammoniumsulfat, behandelt, die ausgefällte Immunglobulin G-hältige Fraktion gelöst und gereinigt wird, wobei vorteilhaft die Reinigung der Immunglobulin G-hältigen Fraktion durchgeführt wird, indem die Lösung über ein Trägermedium geleitet wird, an welchem gereinigtes Flagella-Antigen in monomerer oder polymerer Form kovalent gebunden ist, die flagellenspezifischen Antikörper durch pH-Änderung oder chaotrope Agentien, wie $NH_4SCN$, vom Gel eluiert und in eine galenische Zubereitung formuliert werden.

Die Erfindung wird durch folgende Beispiele näher erläutert, wobei die Beispiele 1 bis 3 die Herstellung von Vakzinen und Beispiel 4 die Herstellung von Immunglobulin G-hältigen Präparationen veranschaulichen.

Beispiel 1:
Vakzine-Formulierung für eine Pseudomonas aeruginosa M-2 Flagellenvakzine

Eine aus Bakterium Pseudomonas aeruginosa M-2-Kultur gewonnene und in oben beschriebener Weise durch Detergensbehandlung, Scheren und chromatographische Behandlung gereinigte Flagellenlösung wurde mit destilliertem pyrogenfreiem Wasser auf eine Proteinkonzentration von 100 $\mu$g/ml eingestellt und durch Zugabe von festem Natriumchlorid und Merthiolat (Endkonzentration 0,9 % NaCl bzw. 0,01 % Merthiolat) isoton gemacht. Anschließend wurde die Lösung durch Sartorius-Einmalfilter (0,2 $\mu$m) sterilfiltriert.

Nach einer Proteinbestimmung der sterilfiltrierten Probe wurde die Flagellensuspension mit steriler NaCl/Merthiolatlösung (0,9 %/0,01 %) auf 25 $\mu$g Protein/ml eingestellt und anschließend durch Zugabe einer 1 % Aluminiumhydroxidsuspension in steriler NaCl/Merthiolatlösung auf 20 $\mu$g Protein/ml verdünnt.

Die fertig adjuvantierte Vakzine enthielt pro ml:

| | |
|---|---|
| 20 $\mu$g | Flagellenprotein |
| 2 mg | $Al(OH)_3$ |
| 9 mg | NaCl |
| 0,1 mg | Merthiolat als Stabilisator. |

Beispiel 2:
Vakzine-Formulierung für eine Pseudomonas aeruginosa 1210 Flagellenvakzine

Eine aus Bakterium Pseudomonas aeruginosa 1210-Kultur gewonnene und in oben beschriebener Weise durch Detergensbehandlung, Scheren und chromatographische Behandlung gereinigte Flagellenlösung wurde mit destilliertem pyrogenfreiem Wasser auf eine Proteinkonzentration von 100 $\mu$g/ml eingestellt und durch Zugabe von festem Natriumchlorid und Merthiolat (Endkonzentration 0,9 % NaCl bzw. 0,01 % Merthiolat) isoton gemacht. Anschließend wurde die Lösung durch Sartorius-Einmalfilter (0,2 $\mu$m) sterilfiltriert.

Nach einer Proteinbestimmung der sterilfiltrierten Probe wurde die Flagellensuspension mit steriler NaCl/Merthiolatlösung (0,9 %/0,01 %) auf 25 $\mu$g Protein/ml eingestellt und anschließend durch Zugabe einer 1 % Aluminiumhydroxidsuspension in steriler NaCl/Merthiolatlösung auf 20 $\mu$g Protein/ml verdünnt.

Die fertig adjuvantierte Vakzine enthielt somit pro ml:

| 20 $\mu$g | Flagellenprotein |
| 2 mg | Al(OH)$_3$ |
| 9 mg | NaCl |
| 0,1 mg | Merthiolat als Stabilisator |

Beispiel 3:
Vakzine-Formulierung für eine Pseudomonas aeruginosa polyvalente Flagellenvakzine

Aus Bakterien Pseudomonas aeruginosa-Kulturen der Typen M-2, 1210, 5142, 5939, 5940 und 170018 gewonnene, in oben beschriebener Weise durch Detergensbehandlung, Scheren und chromatographische Behandlung gereinigte Flagellenlösungen wurden mit destilliertem pyrogenfreiem Wasser auf eine Protein-konzentration von jeweils 100 $\mu$g/ml eingestellt, zu gleichen Teilen vermischt und durch Zugabe von festem Natriumchlorid und Merthiolat (Endkonzentration 0,9 % NaCl bzw. 0,01 % Merthiolat) isoton gemacht. Anschließend wurde durch Sartorius-Einmalfilter (0,2 $\mu$m) sterilfiltriert.

Nach einer Proteinbestimung der sterilfiltrierten Probe wurde die Flagellensuspension mit steriler NaCl/Merthiolatlösung (0,9 %/0,01 %) auf 25 $\mu$g Protein/ml eingestellt und anschließend durch Zugabe einer 1 % Aluminiumhydroxidsuspension in steriler NaCl/Merthiolatlösung auf 20 $\mu$g Protein/ml verdünnt.

Die fertig adjuvantierte Vakzine enthielt somit pro ml:

| 20 $\mu$g | Flagellenproteine |
| 2 mg | Al(OH)$_3$ |
| 9 mg | NaCl |
| 0,1 mg | Merthiolat als Stabilisator |

Beispiel 4:
Pyrogentest

In beschriebener Weise bereitete Vakzine wurde auf das Freisein von pyrogenen Bestandteilen geprüft. Das Versuchsprinzip besteht darin, daß nach der i.v.-Injektion des Produktes in drei oder mehr gesunde, ausgewachsene Kaninchen drei Stunden lang die Temperatur gemessen wird (Eur. Ph., 2nd Ed., 1980, Part 1, V.2.1.4.); der maximale Temperaturanstieg wird für jedes Kaninchen aufgezeichnet und gilt als ein Maß für die Pyrogenität des Produktes.

Für die Durchführung des Versuches wurden verwendet:
Thermoden (Cu-Konstantan Thermoelemente für Kaninchen, Ph-Schenk); Temperaturschreiber (Sechsfarbenpunktdrucker Type STD 62A, Meßbereich 36 bis 42°C, Meßgenauigkeit ±0,08°C); sterile, pyrogenfreie Plastikinjektionsspritzen; sterile, pyrogenfreie Injektionsnadel; Stahlpyrogentestbox mit Halsfixierung; Wagen für sechs Pyrogentestboxen; Wasserbad mit Thermostat; Thermometer; Waage: Sauter ES 120.

Als Versuchstiere wurden gesunde, ausgewachsene Kaninchen beiderlei Geschlechts eingesetzt, die mindestens 1500 g wogen, mit einer vollwertigen antibiotikafreien Standarddiät gefüttert wurden und in der dem Versuch vorausgegangenen Woche keinen Gewichtsabfall zeigten.

Als Probe wurde eine nicht adjuvantierte M2-Flagellenvaccine verwendet und vor der Injektion auf etwa 38°C erhitzt. Der Versuch wurde mit einer Gruppe von drei Kaninchen durchgeführt, die mindestens 90 min vor Testbeginn in die Pyrogentestboxen gebracht wurden.

Für jedes Kaninchen wurde als Anfangstemperatur der Mittelwert aus zwei in einem Abstand von 30 min und in dem Bereich von 40 min vor Verabreichung der Prüflösung erfolgenden Temperaturmessungen bestimmt; Kaninchen, die bei der Bestimmung der Anfangstemperatur zwischen zwei aufeinanderfolgenden Messungen eine größere Temperaturabweichung als 0,2°C zeigten, wurden nicht für den nachfolgenden Versuch eingesetzt. Für die Prüfung wurden nur Kaninchen eingesetzt, die sich in ihrer Anfangstemperatur um nicht mehr als 0,1°C unterschieden. Alle Karinchen, die eine Anfangstemperatur hatten, die höher als 39,8°C oder niedriger als 38,0°C lag, wurden nicht im Hauptversuch eingesetzt. Es wurden 1 ml der Probe/kg Körpergewicht langsam in die Ohrrandvene eines jeden Kaninchens injiziert. Die Injektionsdauer betrug höchstens 4 min. Als Maximaltemperatur wurde für jedes Kaninchen die höchste registrierte Temperatur in drei Stunden nach der Injektion bestimmt. Die Temperatur eines jeden Kaninchens wurde in Abständen von höchstens 30 min aufgezeichnet, beginnend mindestens 90 min vor Verabreichung der Prüfsubstanz und endend drei Stunden nach der Injektion. Die Differenz zwischen der Anfangstemperatur und der Maximaltemperatur eines jeden Kaninchens wurde als Ergebnis gewertet. Negative Differenzen wurden als Null gewertet.

Auswertung:

Zur Auswertung wurden die Temperaturdifferenzen summiert. Bei einer Gruppe von drei Kaninchen entspricht die Probe den Anforderungen, wenn die Summe der Einzelwerte kleiner als 1,15°C ist. Ist die Summe größer als 2,65°C, so entspricht die Probe nicht den Anforderungen.

Bei Ergebnissen, die zwischen den beiden oben genannten Werten liegen, muß der Test wie oben beschrieben wiederholt werden. Es wurden maximal drei Wiederholungen durchgeführt.

Zur Auswertung nach Wiederholungen wurden alle getesteten Tiere (3, 6, 9 oder maximal 12) herangezogen. Die Prüfkriterien sind in der folgenden Tabelle enthalten:

| Anzahl Kaninchen | Die Substanz entspricht der Prüfung auf Pyrogenfreiheit, wenn die Summe der Einzelwerte kleiner ist als | Die Substanz entspricht nicht der Prüfung, wenn die Summe der Einzelwerte größer ist als |
|---|---|---|
| 3 | 1,15° | 2,65° |
| 6 | 2,80° | 4,30° |
| 9 | 4,45° | 5,59° |
| 12 | 6,60° | 6,60° |

Die Probe entspricht den Prüfungserfordernissen, wenn die Summe der drei Einzelwerte kleiner als 1,15°C ist, oder bei Wiederholung die in der obigen Tabelle angeführten Bedingungen erfüllt.

Die erhaltenen Ergebnisse bei dem geprüften M2-Flagellenimpfstoff nach Injektion von 1 ml/kg Kaninchen bei individuellen $\Delta$t-Werten von drei Kaninchen waren die folgenden:

| Impfstoffkonzentration | | |
|---|---|---|
| 10 $\mu$g/ml | 0,1° 0,1° 0,3° | $\Sigma \Delta$t 0,5°C |
| 10 $\mu$g/ml | 0,3° 0,4° 0,2° | $\Sigma \Delta$t 0,9°C |
| 20 $\mu$g/ml | 0,3° 0,5° 0,2° | $\Sigma \Delta$t 1,0°C |
| 20 $\mu$g/ml | 0,2° 0,1° 0,2° | $\Sigma \Delta$t 0,5°C |
| 20 $\mu$g/ml | 0,1° 0,4° 0,2° | $\Sigma \Delta$t 0,7°C |

Die geprüfte Präparation ist somit als frei von pyrogenen Substanzen anzusprechen.

Beispiel 5:
Isolierung der IgG-Fraktion aus Maus-Hyperimmunplasma zur Verwendung bei passiver Immunisierung

Plasma von mit protektivem Flagella(H)-Antigen des Typs M-2 immunisierten Mäusen wurde gesammelt, 30 min bei 56°C unter Schütteln erhitzt, um Fibrin auszufällen. Nach Zentrifugieren während 30 min bei 10.000 x g wurde durch Zugabe von festem Ammoniumsulfat bis zu einer Konzentration von 25 % (w/v) eine Fällung durchgeführt. Die Suspension wurde 20 min gerührt und 1 Stunde bei Raumtemperatur stehengelassen. Hierauf wurde die Suspension während 30 min bei 10.000 x g zentrifugiert und einmal mit gesättigter Ammoniumsulfatlösung gewaschen. Die Fällung wurde in 10 mM Na-Phosphat-Puffer, pH-Wert 7,5, 50 mM NaCl, gelöst und anschließend über Nacht gegen diesen Puffer dialysiert. Nach Zentrifugation wurde eine Ionenaustauschchromatographie (DE-52 Cellulose (Whatman)) durchgeführt. Der Durchlauf enthielt Immunglobulin-G (IgG). Nach Austestung auf IgG wurde die Lösung auf das Ausgangsvolumen mit einer Amicon-Zelle konzentriert und über Nacht gegen eine 0,9 %ige NaCl-Lösung dialysiert. Anschließend wurde eine Gelchromatographie mit Sephadex G-200 (Warenzeichen) durchgeführt. Die IgG-positiven Fraktionen wurden unter Verwendung einer Amicon-Zelle auf das Ausgangsvolumen aufkonzentriert.

Dieses Verfahren stellt eine Modifikation der von D.M.Weir in Edition Handbook of Experimental Immunology, 3rd Edition 1978, Chapter VII, VIII beschriebenen Methode dar.

Im Rahmen der Herstellung von gegen Flagella Antigene der Bakterium Pseudomonas aeruginosa monospezifisch wirksamen Immunglobuline können jedoch auch andere bekannte Methoden außer der Ammonsulfatfällung herangezogen werden, z.B. eine DEAE-Chromatographie, eine Protein A-Affinitätschromatographie oder kombinierte Methoden, die dem Fachmann bekannt sind. Die Immunglobulin G-Präparationen können durch immunaffinitätschromatographische Arbeitsweisen zu monospezifischen Immunglobulinen wieder weitergereinigt werden.

Für die Präparation der monospezifischen Immunglobuline wird mit Vorteil als Immunosorbens ein Trägermedium - vorzugsweise Sepharose 4B Warenzeichen - verwendet, woran gereinigtes Flagellenantigen in monomerer oder polymerer Form kovalent gebunden ist.

Die IgG-hältige Lösung kann sodann mit einer Flußrate von vorzugsweise 1 bis 2 Säulenvolumina pro Stunde über das Affinitätsharz gepumpt werden. Nach dem Waschen des Harzes mit Pufferlösungen höherer Ionenstärken (z.B. 0,5 M NaCl) können die flagellenspezifischen Antikörper durch pH-Änderung oder chaotrope Agentien (z.B. 3M $NH_4SCN$) vom Gel eluiert werden.

Die Wirksamkeit von erfindungsgemäßen protektiven Antigenen und Antikörpern ist im folgenden dargestellt:

Der Nachweis der protektiven Wirksamkeit von erfindungsgemäß für Vakzine zu verwendenden Antigenen erfolgt durch den Nachweis von Flagella(H)-Antikörpern im Serum von Personen, die eine Infektion mit Pseudomonas aeruginosa erlitten haben.

Zur Konzentrationsbestimmung von anti-Flagella Antikörpern wird ein Immunabsorbent Assay, u.zw. ein "Enzyme Linked Immunoabsorbent Assay" (ELISA) oder Radio Immunoassay (RIA) verwendet. Die erfindungsgemäß beschriebenen, hochgereinigten Flagella(H)-Antigene werden mit einem starken Detergens depolymerisiert, anschließend über unspezifische Wechselwirkung an eine Kunststoffoberfläche gebunden und mit Proben von Humanserum in Kontakt gebracht. Befinden sich im Humanserum anti-Flagella(H)-Antikörper, so binden diese spezifisch an das auf der festen Phase immobilisierte Antigen. Mit einem markierten anti-HumanIgG-Antikörper werden auf der Kunststoffoberfläche gebildete Flagella(H)-Antigen-Antikörperkomplexe nachgewiesen.

Dieses Verfahren läßt sich leicht quantitativ auswerten. Tabelle 3 zeigt die Ergebnisse eines Screenings von Serumproben auf Antikörper gegen verschiedene Flagella-Serotypen.

**Tabelle 3**

**Flagella(H)-Antikörpertiter in Humanseren**

| Pseudomonas aeruginosa-Stamm | M2 | 1210 | 5939 | 5940 | 5142 | 170018 |
|---|---|---|---|---|---|---|
| H-Serotyp | b | a0a1a2 | a0a3 | a0a2 | a0 | a0a3a4 |
| Serum # | | | | | | |
| 1 | - | - | + | - | - | + |
| 2 | (+) | (+) | +++ | + | + | +++ |
| 3 | ++ | ++++ | +++ | ++++ | ++ | +++ |
| 4 | - | - | - | - | - | - |
| 5 | + | (+) | ++ | + | - | + |
| 6 | ++ | - | (+) | - | + | - |
| 7 | - | - | - | - | - | - |
| 8 | - | - | ++ | - | - | - |
| 9 | - | - | - | - | - | ++ |
| 10 | - | + | + | + | - | - |
| 11 | - | - | - | - | - | + |
| 12 | - | - | - | - | - | - |
| 13 | - | + | (+) | (+) | - | - |
| 14 | + | - | - | - | + | ++ |
| 15 | + | + | + | + | + | ++ |

- keine Antikörper nachweisbar
(+) knapp über der Nachweisgrenze
+ Titer >1 : 20 >1 : 100
++ Titer >1 : 100
+++ Titer >1 : 200
++++ Titer >1 : 300

Durch die in Tabelle 3 angeführten Ergebnisse über die Auffindung von Flagella(H)-Antikörpern in Seren von nach dem Zufallsprinzip ausgewählten Spendern ist die Immunogenität von nativen Pseudomonas aeruginosa Flagella(H)-Antigenen demonstriert.

Antikörper initiieren die Abwehr von in den Körper eingedrungenen Fremdorganismen in verschiedener Weise. Zusammen mit dem humoralen Abwehrsystem (Komplementsystem) werden von Antikörpern erkannte Fremdorganismen in einer Aufeinanderfolge von Reaktionen durch Lyse abgetötet. Weiterhin können an den Fremdorganismus gebundene Antikörper im Zusammenwirken mit Bestandteilen des Komplementsystems die Aufnahme (Phagocytose) des Fremdorganismus in die Abwehrzellen des Immunsystems (z.B. Granulocyten) induzieren. Im Inneren der Granulocyten kommt es üblicherweise zu einer Abtötung der phagocytierten Fremdzelle.

Im Falle flagellentragender Bakterien kann eine Schutzwirkung von Antikörpern, die gegen den Bewegungsapparat der Zelle gerichtet sind, auch auf die Hemmung der Motilität und damit der Ausbreitung eines Infektionsherdes zurückgeführt werden.

Die Motilitätshemmung durch Flagella(H)-Antikörper kann dadurch demonstriert werden, daß Flagella-(H)-Antigene der in Tabelle 1 aufgeführten Serotypen zur Immunisierung von Mäusen verwendet und das so gewonnene anti-Flagella(H)-Serum bei Motilitäts-Hemmversuchen eingesetzt wird. Mit Antiserum getränkte Filterpapierringe werden auf Motilitäts-Agarplatten gelegt und der Agar innerhalb dieser Ringe mit einer Suspension des homologen Pseudomonasstammes beimpft. Die Zellmenge, die nach 24 h Inkubation außerhalb des Filterpapierringes sichtbar wird, dient als Maß für die Beurteilung der Motilitätshemmung. Die Ergebnisse der Tabelle 4 zeigen, daß mit Flagella(H)-Antigenen gewonnene Maus-Antisera die Motilität des homologen Pseudomonas Bakterienstammes hemmen.

## Tabelle 4

### Motilitätshemmung bei verschiedenen Mengen an homologem Antiflagellenserum

| Stamm | homologes Antiflagellenserum (µl Serum/µl Keimsuspension*) | | | |
|---|---|---|---|---|
| | 30/5 | 20/10 | 10/10 | 5/10 |
| M-2 | - | - | +- | +- |
| 1210 | - | + | + | + |
| 5939 | - | - | - | - |
| 5940 | - | - | - | - |
| 5142 | - | +- | +- | +- |
| 170018 | - | +- | +- | +- |

*) Keimsuspension $10^3$ Keime/ml

- keine Zellen außerhalb des Filterpapierringes

+- Zellen nur an einigen Stellen außerhalb des Filterpapierringes

+ geringes Zellwachstum außerhalb des Filterpapierringes

++ deutliches Zellwachstum außerhalb des Filterpapierringes

mit normalem Mausserum immer deutliches Zellwachstum (++)

Durch die Hemmung der Motilität der in einem Infektionsherd befindlichen Bakterien wird zwar deren weitere Ausbreitung nach der Kolonisierung im Körper des Patienten verhindert, die im Herd befindlichen Bakterien bleiben jedoch weiterhin in der Lage, den Organismus des Patienten durch Abgabe z.B. von Toxinen, Proteasen, etc. zu belasten. Ein echter Schutz gegen Infektion ist nur dann gewährleistet, wenn die bei der aktiven Immunisierung gebildeten Antikörper zu einer schnellen Abtötung der eingedrungenen Bakterien führen.

Im folgenden wird der Nachweis geliefert, daß Flagella(H)-Antikörper Phagocytose und intrazelluläre Abtötung des homologen Pseudomonas Bakterienstammes induzieren.

Zum Phagocytosetest wurden ein in Tabelle 3 nicht aufgeführtes Serum (# 16) sowie die Sera # 3 und # 5 mit einer flagellenlosen (fla⁻) Mutante des Pseudomonas aeruginosa Stammes M-2 (Montie, T.C., D. Doyle-Huntzinger, R.C. Craven, and I.A. Holder (1982) Infect. Immun. 38, 1296-1298) vorinkubiert, um alle nicht Flagellaspezifischen Antikörper aus den Sera zu entfernen. Isolierte Humangranulocyten wurden zur Bestimmung der intrazellulären Abtötung von Pseudomonas aeruginosa M-2 und der isogenen flagellenlosen Mutante in unbehandeltem und in vorabsorbiertem Serum eingesetzt. Die in Tabelle 5 dargestellten Ergebnisse zeigen, daß Flagella(H)-Antikörper die Phagocytose und die darauffolgende intrazelluläre Abtötung des flagellentragenden Stammes von Pseudomonas aeruginosa induzieren, und damit im Sinne der Erfindung eine Schutzwirkung gegen Infektionen mit diesem Keim haben.

## Tabelle 5

Phagocytose und intrazelluläre Abtötung von Pseudomonas aeruginosa M-2 und einer isogenen flagellenlosen Mutante

| Serum# | Verdünnung | Intrazelluläre Abtötung Pseudomonas aeruginosa M-2 | | | Pseudomonas aeruginosa M-2 fla⁻ | | |
|--------|------------|--------|-----------|-----------------------|--------|-----------|-----------------------|
| | | Serum | Serum abs. | Serum ohne Granulocyten | Serum | Serum abs. | Serum ohne Granulocyten |
| 3 | 1 : 25 | 94 % | 59 % | n.b. | 92 % | 0 % | n.b. |
| | 1 : 100 | 52 % | 24 % | n.b. | 36 % | 0 % | n.b. |
| | 1 : 250 | 19 % | 13 % | n.b. | 16 % | 3 % | n.b. |
| 5 | 1 : 25 | 95 % | 51 % | 0 % | 92 % | 1 % | 0 % |
| | 1 : 100 | 72 % | 29 % | 0 % | 58 % | 2 % | 0 % |
| | 1 : 250 | 35 % | 25 % | 0 % | 21 % | 0 % | 0 % |
| 16 | 1 : 25 | 95 % | 57 % | 0 % | 93 % | 0 % | 0 % |
| | 1 : 100 | 78 % | 24 % | n.b. | 70 % | 0 % | n.b. |
| | 1 : 250 | 5 % | 0 % | n.b. | 10 % | 0 % | n.b. |

Der direkte Nachweis der Schutzwirkung von Antigenen, die zu aktiver Immunisierung verwendet werden sollen, kann nur im Tiermodell geführt werden. Um die Situationen zu simulieren, die Patienten für Pseudomonas-Infektionen prädisponieren, fanden zwei in der Literatur beschriebene Tiermodelle Verwendung: das burned mouse Modell (Stieritz, D.D , and I.A. Holder (1975) J. Infect. Dis. 131, 688-691) und das Endoxan Modell (Cryz, S.J.Jr., Fürer, E., Germanier, R. (1983) Infect. Immunity 40, 659-664).

Für beide Modelle wurden die $LD_{50}$ Dosis von Pseudomonas aeruginosa in nicht immunisierten Tieren ermittelt, die im Falle des Endoxan Modelles immunsupprimiert wurden; beim burned mouse Modell wurde eine Verbrennung definierter Größe gesetzt. Nach der Immunisierung mit den in Tabelle 6 angeführten Flagella(H)-Antigenen wurden Tiere mit Verbrennungen bzw. immunsupprimierte Tiere mit einem Vielfachen der vorher ermittelten $LD_{50}$ Keimzahl des homologen Pseudomonas-Stammes infiziert. Tabelle 6 zeigt die Vielfachen der $LD_{50}$ Keimzahlen, die von 100 % der mit der angegebenen Konzentration von Antigen immunisierten Tiere im Endoxan- und im burned mouse-Modell toleriert werden.

Tabelle 7 zeigt die Abhängigkeit der Schutzwirkung des M-2 Flagella-Antigens. In diesem Fall wurden die Tiere mit steigenden Mengen von M-2 Antigen immunisiert und in beiden Modellen getestet. In beiden Fällen ist eine eindeutige Dosisabhängigkeit der Schutzwirkung des zur Immunisierung verwendeten M-2 Flagella-Antigens zu erkennen.

## Tabelle 6

### Aktive Schutzversuche mit Pseudomonas aeruginosa Flagelle(H)-Antigenen

| Antigen | Menge/Maus adjuvantiert mit AL(OH)$_3$ | Immunisierung Tage vor Chall. | Endoxanmodell x LD$_{50}$ | Burned mouse Modell x LD$_{50}$ |
|---|---|---|---|---|
| M-2 | 1 µg | 14 | 10 | $>10^3$ |
|  | 3 µg | 14 | 30 | $>10^4$ |
|  | 3 µg | 14 + 7 | 30 | $>10^4$ |
| 1210 | 3 µg | 14 + 7 | 30 | $>10^4$ |
| M-2 + 1210 | je 1,5 µg | 14 | $10^2$ M-2<br>10   1210 | $>10^4$ M-2<br>$>10^4$ 1210 |
| Polyvalent (6 Typen) | je 3 µg | 14 + 7 | $10^2$ M-2<br>10   1210 | $10^4$ M-2<br>$10^4$ 1210 |

EP 0 252 064 B1

Tabelle 7

Dosisabhängigkeit der Schutzwirkung des M-2 Flagella(H)-Antigens

Zahl der überlebenden Tiere: a) Endoxanmodell b) burned mouse Modell

| Zahl der Challenge-Keime (x LD$_{50}$) | 3 µg | | 1 µg | | 0,3 µg | | 0,1 µg | | 0,03 µg | | 0,01 µg | |
|---|---|---|---|---|---|---|---|---|---|---|---|---|
| | a) | b) | a) | b) | a) | b) | a) | b) | a) | b) | a) | b) |
| 10 | 100% | 80% | 89% | 90% | 100% | 90% | 89% | 40% | 100% | 60% | 70% | 78% |
| 30 | 100% | 80% | 100% | 30% | 90% | 20% | 80% | 10% | 50% | 20% | 40% | 20% |
| 100 | 80% | 60% | 90% | 20% | 90% | 10% | 90% | 0% | 100% | 0% | 30% | 20% |
| 300 | 90% | 50% | 90% | 10% | 100% | 0% | 90% | 10% | 80% | 20% | 20% | 0% |
| 1000 | 100% | 30% | 100% | 20% | 70% | 0% | 100% | 10% | 90% | 20% | 20% | 0% |

Der direkte Nachweis der Schutzwirkung von Antikörpern, die zu passiver Immunisierung verwendet werden sollen, kann nur im Tiermodell geführt werden. Um eine der Situationen zu simulieren, die Patienten für Pseudomonas-Infektionen prädisponieren, fand das in der Literatur beschriebene Endoxan-Modell (Cryz, S.J.Jr., Fürer, E., Germanier, R. 1983: Passive Protection Against Pseudomonas aeruginosa Infection in an Experimental Leukopenic Mouse Model. Infect. Immunity 40, 659-664) Verwendung.

In diesem Test wird die $LD_{50}$ Dosis von Pseudomonas aeruginosa Keimen in immunsupprimierten Tieren ermittelt;-im Falle des hier beschriebenen Experiments lag diese bei $9,5 \times 10^2$ Keimen. Zwei Stunden nach der passiven Immunisierung mit einem Maus anti-Pseudomonas aeruginosa M-2 Hyperimmunplasma, für das im Antikörper-ELISA eine Titerstufe von 1 : 1600 bestimmt wurde, werden die immunsupprimierten Tiere mit einem Vielfachen der vorher ermittelten $LD_{50}$-Keimzahl des homologen Pseudomonas-Stammes infiziert.

Tabelle 8 zeigt, daß passiv immunisierte Tiere bis zu einer 30-fachen $LD_{50}$ Dosis des Challenge Keims geschützt sind.

Tabelle 8

Passiver Pseudomonas-Schutzversuch

E n d o x a n m o d e l l

| Inoculum 2 Stunden von In- fektion | Zahl der überlebenden Tiere nach 3 Tagen (Zahl der Tiere im Versuch) | |
| --- | --- | --- |
| | 0,1 ml Kontrollplasma i.v. | 0,1 ml Hyperimmunplasma i.v. |
| 3 $LD_{50}$ von M-2 | 0 (10) | 9 (10) |
| 10 $LD_{50}$ von M-2 | 0 (10) | 8 (10) |
| 30 $LD_{50}$ von M-2 | 0 (10) | 6 (10) |
| 100 $LD_{50}$ von M-2 | 0 (10) | 0 (10) |
| 300 $LD_{50}$ von M-2 | 0 (10) | 0 (10) |

**Patentansprüche**

1. Gegen Pseudomonas aeruginosa Infektionen wirksame Vakzine, dadurch gekennzeichnet, daß sie protektive Flagella(H)-Antigene vom H-Serotyp a bzw. b enthalten, welche aus monomeren Bestandteilen bestehen, wobei jeder monomere Bestandteil

   a) folgende Aminosäuren: Asparaginsäure (Asp), Threonin (Thr), Serin (Ser), Glutaminsäure (Glu), Glycin (Gly), Alanin (Ala), Valin (Val), Isoleucin (Ile), Leucin (Leu), Tyrosin (Tyr), Phenylalanin (Phe), Lysin (Lys), Arginin (Arg) und gegebenenfalls Tryptophan (Trp) und Methionin (Met) enthält,

   b) die N-terminale Aminosäuresequenz Alanin (Ala) - Leucin (Leu) - Threonin (Thr) - Valin (Val) - Asparagin (Asn) - Threonin (Thr) - Asparagin (Asn) - Isoleucin (Ile) - Alanin (Ala) - aufweist,

   c) ein Molekulargewicht zwischen 43.500 und 53.050 aufweist und

   d) frei ist von Prolin, Halb-Cystin und Histidin

und daß sie frei sind von pyrogenen Substanzen, festgestellt durch Pyrogentests mit drei Kaninchen durch i.v.-Injektion von 1 ml Vakzinlösung/kg Körpergewicht mit 20 $\mu$g/ml Protein, wobei die Summe der drei Einzelwerte kleiner als 1,15°C ist.

2.   Vakzine nach Anspruch 1, dadurch gekennzeichnet, daß sie Flagella(H)-Antigene vom H-Serotyp $a_0$, $a_3$ und $a_4$ enthalten, welche Antigene aus monomeren Bestandteilen bestehen, die die Aminosäuren: Asparaginsäure, Threonin, Serin, Glutaminsäure, Glycin, Alanin, Valin, Isoleucin, Leucin, Tyrosin, Phenylalanin, Lysin und Arginin im Verhältnis 64 : 33 : 35 : 42 : 44 : 68 : 29 : 29 : 37 : 3 : 10 : 19 : 15 enthalten und ein Molekulargewicht von 43.500 aufweisen.

3.   Vakzine nach Anspruch 1, dadurch gekennzeichnet, daß sie Flagella(H)-Antigene vom H-Serotyp $a_0$, $a_3$ enthalten, welche Antigene aus monomeren Bestandteilen bestehen, die die Aminosäuren: Asparaginsäure, Threonin, Serin, Glutaminsäure, Glycin, Alanin, Valin, Isoleucin, Leucin, Tyrosin, Phenylalanin, Lysin und Arginin im Verhältnis 69 : 35 : 38 : 44 : 47 : 73 : 30 : 30 : 60 : 3 : 12 : 21 : 16 enthalten und ein Molekulargewicht von 46.700 aufweisen.

4.   Vakzine nach Anspruch 1, dadurch gekennzeichnet, daß sie Flagella(H)-Antigene vom H-Serotyp $a_0$ enthalten, welche Antigene aus monomeren Bestandteilen bestehen, die die Aminosäuren: Asparaginsäure, Threonin, Serin, Glutaminsäure, Glycin, Alanin, Valin, Isoleucin, Leucin, Tyrosin, Phenylalanin, Lysin und Arginin im Verhältnis 74 : 50 : 49 : 49 : 49 : 89 : 37 : 29 : 44 : 5 : 14 : 17 : 16 enthalten und ein Molekulargewicht von 52.720 aufweisen.

5.   Vakzine nach Anspruch 1, dadurch gekennzeichnet, daß sie Flagella(H)-Antigene vom H-Serotyp b enthalten, welche Antigene aus monomeren Bestandteilen bestehen, die die Aminosäuren: Asparaginsäure, Threonin, Serin, Glutaminsäure, Glycin, Alanin, Valin, Isoleucin, Leucin, Tyrosin, Phenylalanin, Lysin und Arginin im Verhältnis 74 : 48 : 48 : 49 : 51 : 91 : 38 : 30 : 43 : 4 : 13 : 18 : 18 enthalten und ein Molekulargewicht von 53.050 aufweisen.

6.   Vakzine nach Anspruch 1, dadurch gekennzeichnet, daß sie Flagella(H)-Antigene vom H-Serotyp $a_0$, $a_1$, $a_2$ enthalten, welche Antigene aus monomeren Bestandteilen bestehen, die die Aminosäuren: Asparaginsäure, Threonin, Serin, Glutaminsäure, Glycin, Alanin, Valin, Isoleucin, Leucin, Tyrosin, Phenylalanin, Lysin und Arginin im Verhältnis 76 : 44 : 40 : 52 : 50 : 81 : 32 : 32 : 41 : 4 : 12 : 20 : 18 enthalten und ein Molekulargewicht von 51.250 aufweisen.

7.   Vakzine nach Anspruch 1, dadurch gekennzeichnet, daß sie Flagella(H)-Antigene vom H-Serotyp $a_0$, $a_2$ enthalten, welche Antigene aus monomeren Bestandteilen bestehen, die die Aminosäuren: Asparaginsäure, Threonin, Serin, Glutaminsäure, Glycin, Alanin, Valin, Isoleucin, Leucin, Tyrosin, Phenylalanin, Lysin und Arginin im Verhältnis 68 : 41 : 37 : 46 : 44 : 73 : 29 : 29 : 37 : 3 : 10 : 16 : 16 enthalten und ein Molekulargewicht von 45.900 aufweisen.

8.   Verfahren zur Herstellung von Vakzinen gemäß den Ansprüchen 1 bis 7, dadurch gekennzeichnet, daß Kulturen von Pseudomonas aeruginosa mit einem Detergens behandelt, einem Scherprozeß unterworfen, um Flagella(H)-Antigene abzutrennen, und das abgetrennte Antigen chromatographisch gereinigt, sterilfiltriert, gegebenenfalls mit einem Adjuvans, wie Al(OH)$_3$ vermischt, gewünschtenfalls mit einem Präservans, wie Merthiolat™, versetzt und in eine galenische Zubereitung formuliert wird.

9.   Immunglobulin G-hältige, gegen Bakterium Pseudmonas aeruginosa-Infektionen wirksame Präparationen zur prophylaktischen und therapeutischen Anwendung, dadurch gekennzeichnet, daß sie aus dem Blutplasma von mit protektiven Flagella(H)-Antigenen gemäß einem der Ansprüche 1 bis 7 immunisierten menschlichen Spendern bzw. Säugetieren gewonnene Flagella(H)-Antikörper enthalten.

**10.** Präparationen nach Anspruch 9, dadurch gekennzeichnet, daß sie Flagella(H)-Antikörper mit einer motilitätshemmenden Wirkung sowie mit der Fähigkeit einer erhöhten Phagocytose und einer erhöhten intrazellulären Abtötung von Pseudomonas aeruginosa-Bakterien aufweisen.

**11.** Verfahren zur Herstellung von Immunglobulin G-hältigen, gegen Bakterium Pseudmonas aeruginosa-Infektionen wirksamen Präparationen gemäß den Ansprüchen 9 und 10, dadurch gekennzeichnet, daß Plasma von mit in den Ansprüchen 1 bis 7 genannten protektiven Flagella(H)-Antigenen immunisierten menschlichen Spendern bzw. Säugetieren mit Proteinfällungsmitteln, wie Ammoniumsulfat, behandelt, die ausgefällte Immunglobulin G-hältige Fraktion gelöst und gereinigt wird.

**12.** Verfahren nach Anspruch 11, dadurch gekennzeichnet, daß die Reinigung der Immunglobulin G-hältigen Fraktion durchgeführt wird, indem die Lösung über ein Trägermedium geleitet wird, an welchem gereinigtes Flagella-Antigen in monomerer oder polymerer Form kovalent gebunden ist, die flagellenspezifischen Antikörper durch pH-Änderung oder chaotrope Agentien, wie $NH_4SCN$, vom Gel eluiert und in eine galenische Zubereitung formuliert werden.

**Claims**

**1.** Vaccines active against Pseudomonas aeruginosa infections, characterised in that they contain protective flagellar (H) antigens of the H-serotype $\underline{a}$ and $\underline{b}$, respectively, which consist of monomeric components, each monomeric component

a) containing the following amino acids: aspartic acid (Asp), threonine (Thr), serine (Ser), glutamic acid (Glu), glycine (Gly), alanine (Ala), valine (Val), isoleucine (Ile), leucine (Leu), tyrosine (Tyr), phenylalanine (Phe), lysine (Lys), arginine (Arg), and optionally tryptophane (Trp) and methionine (Met),

b) having the N-terminal amino acid sequence alanine (Ala) - leucine (Leu) - threonine (Thr) - valine (Val) - asparagin (Asn) - threonine (Thr), asparagine (Asn) - isoleucine (Ile) - alanine (Ala),

c) having a molecular weight between 43,500 and 53,050 and

d) being free from proline, semi-cystine and histidine

and being free from pyrogenic substances, determined by the pyrogen test with three rabbits by i.v. injection of 1 ml vaccine solution/kg body weight with 20 $\mu$g/ml protein, the sum of the three individual values being less than 1.15°C.

**2.** Vaccines according to claim 1, characterised in that they contain flagellar (H) antigens of the H-serotype $a_0$, $a_3$ and $a_4$, which antigens consist of monomeric components containing the amino acids: aspartic acid, threonine, serine, glutamic acid, glycine, alanine, valine, isoleucine, leucine, tyrosine, phenylalanine, lysine and arginine at a ratio of 64 : 33 : 35 : 42 : 44 : 68 : 29 : 29 : 37 : 3 : 10 : 19 : 15 and having a molecular weight of 43,500.

**3.** Vaccines according to claim 1, characterised in that they contain flagellar (H) antigens of the H-serotype $a_0$, $a_3$, which antigens consist of monomeric components containing the amino acids: aspartic acid, threonine, serine, glutamic acid, glycine, alanine, valine, isoleucine, leucine, tyrosine, phenylalanine, lysine and arginine at a ratio of 69 : 35 : 38 : 44 : 47 : 73 : 30 : 30 : 60 : 3 : 12 : 21 : 16 and having a molecular weight of 46,700.

**4.** Vaccines according to claim 1, characterised in that they contain flagellar (H) antigens of the H-serotype $a_0$, which antigens consist of monomeric components containing the amino acids: aspartic acid, threonine, serine, glutamic acid, glycine, alanine, valine, isoleucine, leucine, tyrosine, phenylalanine, lysine and arginine at a ratio of 74 : 50 : 49 : 49 : 49 : 89 : 37 : 29 : 44 : 5 : 14 : 17 : 16 and having a molecular weight of 52,720.

**5.** Vaccines according to claim 1, characterised in that they contain flagellar (H) antigens of the H-serotype $\underline{b}$, which antigens consist of monomeric components containing the amino acids: aspartic acid, threonine, serine, glutamic acid, glycine, alanine, valine, isoleucine, leucine, tyrosine, phenylalanine, lysine and arginine at a ratio of 74 : 48 : 48 : 49 : 51 : 91 : 38 : 30 : 43 : 4 : 13 : 18 : 18 and having a molecular weight of 53,050.

**6.** Vaccines according to claim 1, characterised in that they contain flagellar (H) antigens of the H-

EP 0 252 064 B1

serotype $a_0$, $a_1$, $a_2$, which antigens consist of monomeric components containing the amino acids: aspartic acid, threonine, serine, glutamic acid, glycine, alanine, valine, isoleucine, leucine, tyrosine, phenylalanine, lysine and arginine at a ratio of 76 : 44 : 40 : 52 : 50 : 81 : 32 : 32 : 41 : 4 : 12 : 20 : 18 and having a molecular weight of 51,250.

7. Vaccines according to claim 1, characterised in that they contain flagellar (H) antigens of the H-serotype $a_0$, $a_2$, which antigens consist of monomeric components containing the amino acids: aspartic acid, threonine, serine, glutamic acid, glycine, alanine, valine, isoleucine, leucine, tyrosine, phenylalanine, lysine and arginine at a ratio of 68 : 41 : 37 : 46 : 44 : 73 : 29 : 29 : 37 : 3 : 10 : 16 : 16 and having a molecular weight of 45,900.

8. A method of producing vaccines according to claims 1 to 7, characterised in that cultures of Pseudomonas aeruginosa are treated with a detergent, are subjected to a shearing process to separate flagellar (H) antigens, and that the separated antigen is chromatographically purified, sterile-filtered, possibly is mixed with an adjuvans, such as $Al(OH)_3$, if desired is admixed with a preservative, such as Merthiolat™, and is formulated into a galenic preparation.

9. Immunoglobulin-G-containing preparations active against bacterium Pseudomonas aeruginosa infections for prophylactic and therapeutic applications, characterised in that they comprise flagellar (H) antibodies obtained from blood plasma of human donors or mammals immunized with protective flagellar (H) antigens according to any of claims 1 to 7.

10. Preparations according to claim 9, characterised in that they contain flagellar (H) antibodies having a motility inhibiting activity as well as the capability of an elevated phagocytosis and an elevated intracellular killing of Pseudomonas aeruginosa bacteria.

11. A method of producing immunoglobulin-G-containing preparations active against bacterium Pseudomonas aeruginosa infections according to claims 9 and 10, characterised in that plasma of human donors or mammals immunized with the protective flagellar (H) antigens named in claims 1 to 7 is treated with protein-precipitating agents, such as ammonium sulfate, the precipitated immunoglobulin-G-containing fraction is dissolved and purified.

12. A method according to claim 11, characterised in that the purification of the immunoglobulin-G-containing fraction is carried out by passing the solution over a carrier medium to which purified flagellar antigen in monomeric or polymeric form is covalently bound, the flagellar-specific antibodies are eluted from the gel by pH-change or by chaotropic agents, such as $NH_4SCN$, and are formulated into a galenic preparation.

**Revendications**

1. Vaccins efficaces contre les infections à Pseudomonas aeruginosa, caractérisés en ce qu'ils contiennent des antigènes H flagellaires protecteurs du sérotype H a ou b, constitués par des composants monomères, chaque composant monomère

a) contenant les acides aminés suivants : acide asparagique (Asp), thréonine (Thr), sérine (Ser), acide glutamique (Glu), glycine (Gly), alanine (Ala), valine (Val), isoleucine (Ile), leucine (Leu), tyrosine (Tyr), phénylalanine (Phe), lysine (Lys), arginine (Arg) et le cas échéant typtophane (Trp) et méthionine (Met),

b) présentant la séquence d'acides aminés N terminale alanine (Ala) - leucine (Leu) - thréonine (Thr) - valine (Val) - asparagine (Asn) - thréonine (Thr) - asparagine (Asn) - isoleucine (Ile) - alanine (Ala) -,

c) présentant un poids moléculaire compris entre 43 500 et 53 050 et

d) étant exempt de proline, d'hémicystine et d'histidine

et en ce qu'ils sont exempts de substances pyrogènes, ce qui est constaté par le test des pyrogènes chez trois lapins par injection i.v. de 1 ml de solution de vaccin par kg de poids corporel contenant 20 $\mu$g/ml de protéines, la somme des trois résultats individuels étant inférieure à 1,15°C

2. Vaccins selon la revendication 1, caractérisés en ce qu'ils contiennent des antigènes H de flagelles du sérotype H $a_0$, $a_3$, $a_4$, lesquels antigènes sont constitués par des composants monomères qui contiennent les acides aminés : acide asparagique, thréonine, sérine, acide glutamique, glycine,

21

EP 0 252 064 B1

alanine, valine, isoleucine, leucine, tyrosine, phénylalanine, lysine et arginine dans le rapport de 64 : 33 : 35 : 42 : 44 : 68 : 29 : 29 : 37 : 3 : 10 : 19 : 15 et présentent un poids moléculaire de 43 500.

3. Vaccins selon la revendication 1, caractérisés en ce qu'ils contiennent des antigènes H de flagelles du sérotype H $a_0$, $a_3$, lesquels antigènes sont constitués par des composants monomères qui contiennent les acides aminés : acide asparagique, thréonine, sérine, acide glutamique, glycine, alanine, valine, isoleucine, leucine, tyrosine, phénylalanine, lysine et arginine dans le rapport de 69 : 35 : 38 : 44 : 47 : 73 : 30 : 30 : 60 : 3 : 12 : 21 : 16 et présentent un poids moléculaire de 46 700.

4. Vaccins selon la revendication 1, caractérisés en ce qu'ils contiennent des antigènes H de flagelles du sérotype H $a_0$, lesquels antigènes sont constitués par des composants monomères qui contiennent les acides aminés : acide asparagique, thréonine, sérine, acide glutamique, glycine, alanine, valine, isoleucine, leucine, tyrosine, phénylalanine, lysine et arginine dans le rapport de 74 : 50 : 49 : 49 : 49 : 89 : 37 : 29 : 44 : 5 : 14 : 17 : 16 et présentent un poids moléculaire de 52 720.

5. Vaccins selon la revendication 1, caractérisés en ce qu'ils contiennent des antigènes de flagelles du sérotype $H_b$, lesquels antigènes sont constitués par des composants monomères qui contiennent les acides aminés : acide asparagique, thréonine, sérine, acide glutamique, glycine, alanine, valine, isoleucine, leucine, tyrosine, phénylalanine, lysine et arginine dans le rapport de 74 : 48 : 48 : 49 : 51 : 91 : 38 : 30 : 43 : 4 : 13 : 18 : 18 et présentent un poids moléculaire de 53 050.

6. Vaccins selon la revendication 1, caractérisés en ce qu'ils contiennent des antigènes H de flagelles du sérotype H $a_0$, $a_1$, $a_2$, lesquels antigènes sont constitués par des composants monomères qui contiennent les acides aminés : acide aspa-ragique, thréonine, serine, acide glutamique, glycine, alanine, valine, isoleucine, leucine, tyrosine, phénylalanine, lysine et arginine dans le rapport de 76 : 44 : 40 : 52 : 50 : 81 : 32 : 32 : 41 : 4 : 12 : 20 : 18 et présentent un poids moléculaire de 51 250.

7. Vaccins selon la revendication 1, caractérisés en ce qu'ils contiennent des antigènes H de flagelles du sérotype H $a_0$, $a_2$, lesquels antigènes sont constitués par des composants monomères qui contiennent les acides aminés : acide asparagique, thréonine, sérine, acide glutamique, glycine, alanine, valine, isoleucine, leucine, tyrosine, phénylalanine, lysine et arginine dans le rapport de 68 : 41 : 37 : 46 : 44 : 73 : 29 : 29 : 37 : 3 : 10 : 16 : 16 et présentent un poids moléculaire de 45 900.

8. Procédé de préparation de vaccins selon les revendications 1 à 7, caractérisé en ce que des cultures de Pseudomonas aeruginosa sont traitées par un détergent, soumises à un processus de cisaillement pour séparer les antigènes H flagellaires et en ce que l'antigène séparé est purifié par chromatographie, filtré stérilement, le cas échéant mélangé à un adjuvant tel que Al(OH)$_3$, qu'il lui est ajouté si nécessaire un agent de préservation tel que merthiolate™ et qu'on le formule sous la forme d'une préparation galénique.

9. Préparations efficaces contre les infections par le bacille Pseudomonas aeruginosa, contenant des immunoglobulines G, destinées à un usage prophylactique et thérapeutique, caractérisées en ce qu'elles contiennent des anticorps anti-flagelles H obtenus à partir du plasma sanguin de donneurs humains ou de mammifères immunisés par des antigènes H flagellaires protecteurs selon l'une des revendications 1 à 7.

10. Préparations selon la revendication 9, caractérisées en ce qu'elles présentent des anticorps anti-flagelles H ayant un effet d'inhibition de la motilité ainsi que la capacité d'accroître la phagocytose et la destruction intracellulaire de bacilles Pseudomonas aeruginosa.

11. Procédé de fabrication de préparations efficaces contre les infections par le bacille Pseudomonas aeruginosa, contenant des immunoglobulines G, selon les revendications 9 et 10, caractérisé en ce que le plasma de donneurs humains ou de mammifères immunisés par des antigènes H flagellaires protecteurs cités dans les revendications 1 à 7 est traité par des agents de précipitation des protéines tels que le sulfate d'ammonium, que la fraction précipitée contenant les immunoglobulines G est dissoute et purifiée.

12. Procédé selon la revendication 11, caractérisé en ce que la purification de la fraction contenant des

22

immunoglobulines G est opérée par envoi de la solution sur un milieu support auquel est fixé par covalence l'antigène flagellaire purifié sous une forme monomère ou polymère, par élution des anticorps spécifiques des flagelles du gel par modification du pH ou par des agents chaotropes tels que $NH_4SCN$, et par formulation sous la forme d'une préparation galénique.